(19) Europäisches Patentamt
European Patent Office
Office européen des brevets

(11) Veröffentlichungsnummer: 0 115 789
B1

(12) # EUROPÄISCHE PATENTSCHRIFT

(45) Veröffentlichungstag der Patentschrift: 28.02.90

(51) Int. Cl. 5: A 61 K 31/415

(21) Anmeldenummer: 84100305.6

(22) Anmeldetag: 13.01.84

(54) Verwendung von "Nafazatrom".

(30) Priorität: 28.01.83 DE 3302811
12.03.83 DE 3308880

(43) Veröffentlichungstag der Anmeldung: 15.08.84 Patentblatt 84/33

(45) Bekanntmachung des Hinweises auf die Patenterteilung: 28.02.90 Patentblatt 90/09

(84) Benannte Vertragsstaaten: AT BE CH DE FR GB IT LI LU NL SE

(56) Entgegenhaltungen:
Naunyn-Schmiedeberg's Arch. Pharmacol., Band 321, Suppl., 1982, Seite R 54, Ref. Nr. 215 V.B. Fiedler: "Evalution of epicardial R wave voltage and myocardial ischemia following experimental coronary rteri thrombosis: Effects of nafazatrom"
Z. Kardiol., Band 71, Nr. 9, 1982, Ref. Nr. P 133 V.B. Fiedler: "Wirkungen von Nafazatrom auf die Koronararterienthrombose und Myokardischämie des narkotisierten Hundes"
Z. Kardiol., Band 71, Nr. 3, 1982, Ref. Nr. P 424 V.B. Fiedler: "Effects of nafazatrom on coronary artery thrombosis and myoardial ischemia"
Fed. Proc., Band 41, 1982, Seite 1717 Ref. Nr.8464, W.D. Busse et al.: "Nafazatrom (BAY q 6575) an inhibitor of cellular lipoxycenase activity"
Die Akte enthält technische Angaben, die nach dem Eingang der Anmeldung eingereicht wurden und die nicht in dieser Patentschrift enthalten sind.

(73) Patentinhaber: BAYER AG
D-5090 Leverkusen 1 Bayerwerk (DE)

(72) Erfinder: Mardin, Mithat, Dr.
Molecular Diagnostics, Inc. 400 Morgan Lane
West Haven Ct. 06516 (US)
Erfinder: Seuter, Friedel, Dr.
Moospfad 16
D-5600 Wuppertal 1 (DE)
Erfinder: Busse, Wolf-Dieter, Dr.
Molecular Diagnostics, Inc. 400 Morgan Lane
West Haven Ct. 06516 (US)
Erfinder: Perzborn, Elisabeth, Dr.
Am Tescherbusch 13
D-5600 Wuppertal 11 (DE)
Erfinder: Hoffmeister, Friedrich, Prof. Dr.
Katernberger Strasse 262
D-5600 Wuppertal 1 (DE)
Erfinder: Fiedler, Volker, Dr.
Kluser Höhe 6
D-5600 Wuppertal 1 (DE)
Erfinder: Schlossmann, Klaus, Dr.
In der Beek 116
D-5600 Wuppertal 1 (DE)
Erfinder: Mayer, Dieter, Prof. Dr.
Beethovenstrasse 1
D-4712 Werne (DE)

EP 0 115 789 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBERGRAF, STOCKHOLM 1990

## Beschreibung

Die vorliegende Erfindung betrifft die Verwendung von 3-Methyl-1-[2-(2-naphthoxy)ethyl]-2-pyrazolin-5-on ("Nafazatrom") zur Herstellung von Arzneimitteln zur Behandlung von asthmatischen Erkrankungen.

Es ist bekannt, daß Nafazatrom starke antithrombotische Wirkung aufweist (DE-AS 2 427 272, US-PS 4 053 621), aber auch zur Verminderung von Metastasenbildung und neoplastischem Wachstum in Säugern eingesetzt werden kann (EP-A. 0 062 319).

Als Antithrombotikum war Nafazatrom im Tierversuch z. B. bei Ratte, Meerschweinchen und Kaninchen arteriell und venös (V. jugularis, A. carotis, A. femoralis) wirksam (Arzneim. Forsch. 29, 54, 1979; Z. Kardiol. Band 71, Nr. 3,1982, Ref. Nr. P424) und auch in den Koronararterien des Hundes (Naunyn-Schmiedeberg's Arch. Pharmacol. Band 321, Suppl. 1982, Seite R54, Ref. Nr. 215 und Z. Kardiol. Band 71, Nr. 9, 1982, Ref. Nr. P 133).

Mehrere Untersuchungen mit Nafazatrom zeigten, daß die Substanzen als Stimulator von endogenem Prostacyclin ($PGI_2$) aus dem Gefäßendothel wirken kann (Lancet 1, 528, 1979; Haemostasis *10*, 297, 1981; Thrombosis and Haemostasis, *46*, Abstr. No. 45, 1981). Von Wong und McGiff (J. Pharmacol. Exp. Ther. *223*, 757 - 760, 1982) wurde gezeigt, daß Nafazatrom die 15-Hydroxyprostaglandindehydrogenase, das $PGI_2$-abbauende Enzym, blockiert.

Darüberhinaus konnte an normalen und neoplastischen Zellen gezeigt werden, daß die bekannte antithrombotische und antimetastasische Wirkung von Nafazatrom auf eine Hemmung der cellulären Lipoxygenase zurückgeführt werden kann (Fed. Proc. Band 41, 1982, Seite 1717, Ref. Nr. 8464).

Es wurde nun die Verwendung von Nafazatrom zur Herstellung eines Antiasthmatikums gefunden.

Aus der Kenntnis des Standes der Technik konnte nicht erwartet werden, daß Nafazatrom eine solche ausgeprägte antiasthmatische Wirkung besitzt.

Überraschenderweise hemmt Nafazatrom selektiv die für die Biosynthese des chemotaktisch wirksamen Leukotriens $B_4$ und der spasmogen wirksamen Leukotriene $C_4$ und $D_4$ verantwortliche 5-Lipoxygenase, während die für die Biosynthese des endogenen Lipoxygenaseinhibitors 15-Hydroxyeikosatetraensäure (15-HETE) zuständige 15-Lipoxygenase nicht gehemmt bzw. bei Konzentrationen >10 µg/ml sogar stimuliert wird. Ebenso wird die 12-Lipoxygenaseaktivität von Nafazatrom bei diesen Konzentrationen nicht gehemmt.

Überraschenderweise kann Nafazatrom daher zur Herstellung eines Antiasthmatikums verwendet werden.

Die bronchodilatorischen Eigenschaften des Nafazatroms, die für Antiasthmatika wesentlich sind, lassen sich z. B. an der isolierten Meerschweinchenlunge zeigen. Nafazatrom hemmt in diesem Organ die Methacholin-induzierte Bronchokonstriktion (Methode nach F.P. Luduena et al. Arch. Int. Pharmacodyn. 111, 392 (1957)).

Das erfindungsgemäß verwendete Nafazatrom (Formel I) ist aus US-P 4 053 621 bekannt und kann auf verschiedenenen Synthesewegen hergestellt werden.

Nach Verfahren A wird 2-(2-Naphthyloxy-ethylhydrazin mit einem Acetessigsäure-Derivate umgesetzt.

Nach Verfahren B wird 3-Methylpyrazolinon-(5) mit einem 2-(2-Naphthyloxy)-ethyl-Derivat umgesetzt. Nach Verfahren C wird 2-(2-Naphthyloxy)-ethylhydrazin mit einem Tetrolsäure-Derivat umgesetzt.

Die Herstellungsvarianten A, B und C können durch das folgende Reaktionsschema erläutert werden:

A.

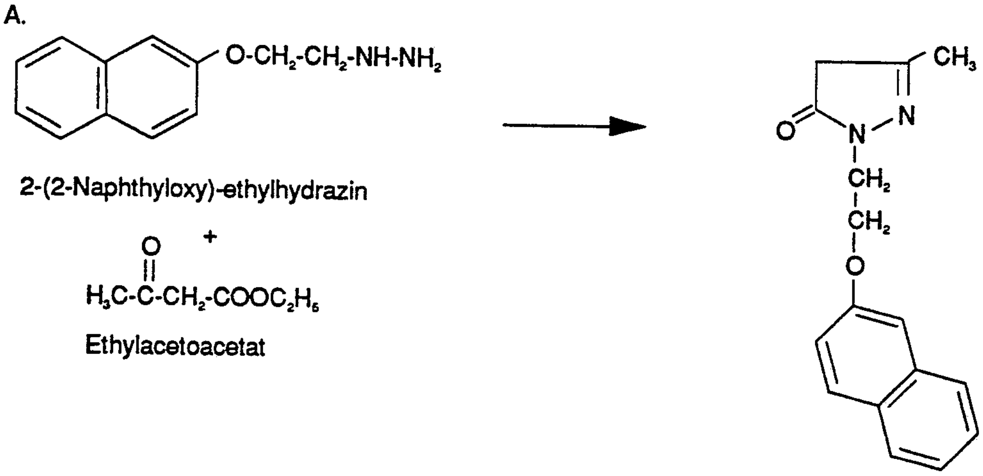

2-(2-Naphthyloxy)-ethylhydrazin

+

Ethylacetoacetat

I

B.

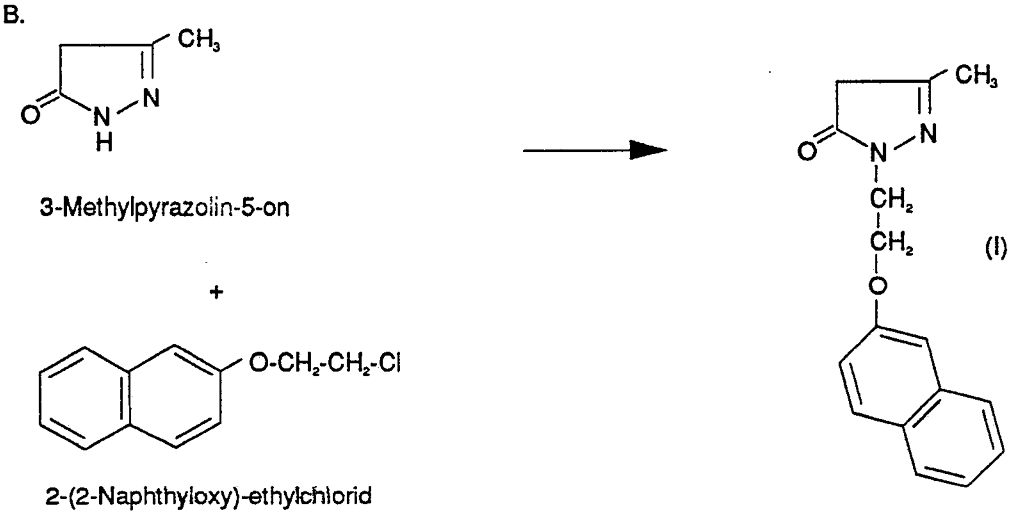

3-Methylpyrazolin-5-on

+

2-(2-Naphthyloxy)-ethylchlorid

(I)

Nafazatrom

C.

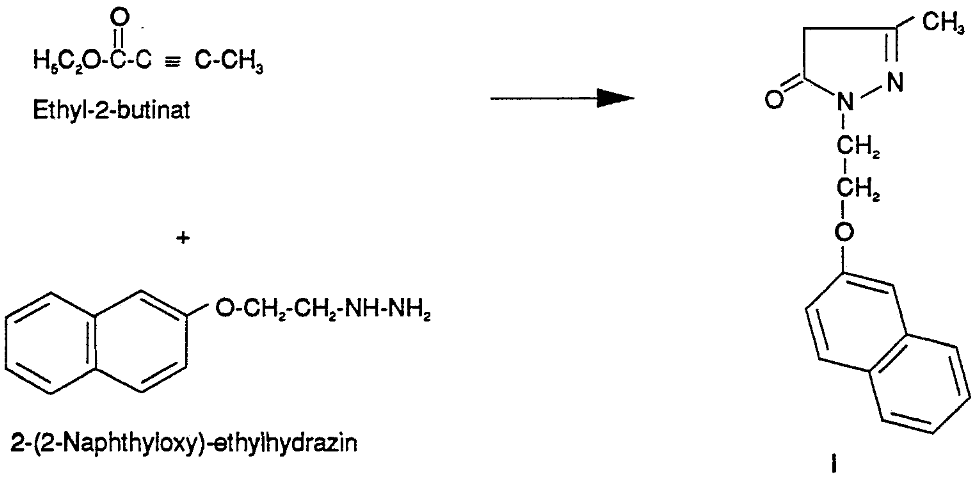

Ethyl-2-butinat

+

2-(2-Naphthyloxy)-ethylhydrazin

I

Als Reaktionsmedium kommen sämtliche inerten organischen Lösungsmittel, wahlweise verdünnt mit Wasser, z.B. Kohlenwasserstoffe wie Benzol und Toluol, Halogenkohlenwasserstoffe wie Methylenchlorid, Alkohole wie Methanol und Ethanol, und organische Basen wie Pyridin und Picolin in Frage.

Für die obigen Verfahren zur Herstellung von Nafazatrom können basische oder saure Kondensationsmittel verwendet werden, und die Reaktionstemperatur kann zwischen 10°C und 200°C variiert werden. Die Verbindung kann in einfacher Weise mittels üblicher Methoden durch Umkristallisieren aus einem geeigneten Lösungsmittel gereinigt werden.

In den Arzneimitteln gemäß vorliegender Erfindung sind neben Nafazatrom auch pharmazeutisch unbedenkliche Verdünnungsmittel oder Trägermaterialien enthalten. Hierunter sind nicht-toxische Substanzen zu verstehen, die nach dem Vermischen mit dem Wirkstoff diesen in einer für die Verabreichung geeigneten Form liefern. Die Bezeichnung schließt vorzugsweise Wasser und üblicherweise bei der chemischen Synthese verwendete organische Lösungsmittel mit niederem Molekulargewicht aus, außer bei Vorliegen anderer pharmazeutisch erforderlicher Bestandteile wie Salze in den richtigen Mengen, um eine isotonische Zubereitung herzustellen, Puffer, oberflächenaktive Mittel, Farb- und Geschmacksstoffe und Konservierungsmittel. Beispiele für geeignete feste und flüssige Verdünnungsmittel und Trägermaterialien sind die folgenden: wasserhaltige Puffermittel, die durch Zusatz von Glucose oder Salzen isotonisch gemacht werden können; nicht-toxische organische Lösungsmittel wie Paraffine, pflanzliche Öle, Alkohole, Glycole; gemahlene Natursteinmaterialien (beispielsweise Kaoline, Aluminiumoxide, Talkum oder Kreide); synthetische Gesteinspulver (beispielsweise hochdisperse Kieselsäure oder Silicate); Zucker; wäßrige Suspensionen von Cellulosederivaten, z.B. Methylhydroxylalkyl-Cellulose (Tylose).

In der Regel enthalten die erfindungsgemäßen Arzneimittel 0,5 - 95 Gew.-%, bevorzugt 1 - 90 Gew.-%, besonders bevorzugt 5 - 50 Gew.-% an Nafazatrom.

Die orale Verabreichung kann unter Verwendung fester und flüssiger Dosierungsformen wie Pulver, Tabletten, Dragees, Kapseln, Granulat, Suspensionen, Lösungen und dergleichen vorgenommen werden. Gegebenenfalls können die Dosierungseinheiten für die orale Verabreichung mikroverkapselt werden, um die Abgabe hinauszuzögern oder über längere Zeit hinweg zu verlangsamen, wie beispielsweise durch Überziehen oder Einbetten von teilchenförmigem Material in Polymere, Wachs oder dergleichen.

Die parenterale Verabreichung kann unter Verwendung flüssiger Dosierungsformen wie steriler Lösungen und Suspensionen erfolgen, die für die subkutane, intramuskuläre oder intravenöse Injektion bestimmt sind. Diese werden durch Suspendieren oder Auflösen einer abgemessenen Menge des Wirkstoffes in einem zur Injektion geeigneten nicht-toxischen flüssigen Streckmittel wie einem wäßrigen oder öligen Medium und Sterilisierung der Suspension oder Lösung hergestellt. Stabilisatoren, Konservierungsmittel und Emulgatoren können ebenfalls zugesetzt werden.

Im allgemeinen beträgt beim Menschen die auf das Körpergewicht bezogene Tagesdosis des Wirkstoffs für die parenterale Verabreichung 0,01 bis 50 mg/kg, vorzugsweise 0,1 bis 10 mg/kg, und für die orale Verabreichung 0,1 bis 500 mg/kg, vorzugsweise 0,5 bis 100 mg/kg, besonders bevorzugt 1 bis 50 mg/kg.

Die Dosierungseinheit (Tablette, Kapsel, etc.) enthält in der Regel 1 - 100 mg, bevorzugt 5 - 50 mg, besonders bevorzugt 10 - 30 mg an Nafazatrom.

**Beispiel**

**Bronchodilatierende Wirkung**

Die Meerschweinchenlunge wird mit einer physiologischen Pufferlösung (Tyrodelösung), die 0,05 µg/ml Methacholin enthält, perfundiert. Durch die erfolgende Bronchokonstriktion wird die Flußrate herabgesetzt und entsprechend der Perfusionsdruck erhöht. Nafazatrom hemmt bei $10^{-4}$ g/ml zu 50 % den Anstieg des Perfusionsdruckes und damit die Bronchokonstriktion.

## Patentansprüche

1. Verwendung von Nafazatrom zur Herstellung von Arzneimitteln zur Behandlung von asthmatischen Erkrankungen.

2. Verwendung von Nafazatrom zur Herstellung von 5-lipoxygenasehemmenden Arzneimitteln zur Behandlung von asthmatischen Erkrankungen.

## Claims

1. Use of nafazatrom for the preparation of medicaments for the treatment of asthmatic diseases.

2. Use of nafazatrom for the preparation of 5-lipoxygenase-inhibiting medicaments for the treatment of asthmatic diseases.

## Revendications

1. Utilisation du Nafazatrom pour la fabrication de médicaments pour le traitement de maladies asthmatiques.

2. Utilisation du Nafazatrom pour la fabrication de médicaments inhibant la 5-lipoxygénase pour le traitement de maladies asthmatiques.